(19) Europäisches Patentamt
European Patent Office
Office européen des brevets

(11) Veröffentlichungsnummer: **0 221 494**
**B1**

(12) **EUROPÄISCHE PATENTSCHRIFT**

(45) Veröffentlichungstag der Patentschrift: **03.08.88**

(51) Int. Cl.[4]: **C 07 C 102/00**, C 07 C 103/34, C 07 D 295/18

(21) Anmeldenummer: **86114932.6**

(22) Anmeldetag: **27.10.86**

(54) **Verfahren zur Herstellung von Glykolsäureamiden.**

(30) Priorität: **07.11.85 DE 3539394**

(43) Veröffentlichungstag der Anmeldung: **13.05.87 Patentblatt 87/20**

(45) Bekanntmachung des Hinweises auf die Patenterteilung: **03.08.88 Patentblatt 88/31**

(84) Benannte Vertragsstaaten: **BE CH DE FR GB IT LI NL**

(56) Entgegenhaltungen:
**EP - A - 0 014 409**
**EP - A - 0 091 851**
**DE - A - 3 038 598**

(73) Patentinhaber: **BAYER AG, Konzernverwaltung RP Patentabteilung, D-5090 Leverkusen 1 Bayerwerk (DE)**

(72) Erfinder: **Diehr, Hans-Joachim, Dr., Höhe 35, D-5600 Wuppertal 11 (DE)**

EP 0 221 494 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft ein neues Verfahren zur Herstellung von bekannten Glykolsäureamiden, die als Zwischenprodukte für die Synthese von herbiziden Wirkstoffen verwendet werden können.

Es ist bereits bekannt, dass sich Glykolsäureamide in einer dreistufigen Reaktionsfolge herstellen lassen, indem man zunächst Chloracetylchlorid mit einem Amin umsetzt, das so erhaltene Chloracetamid in einer 2. Stufe mit einem Alkalicarboxylat zum entsprechenden Acyloxyacetamid umsetzt und dieses in einer 3. Stufe zum gewünschten Glykolsäureamid verseift (vgl. z.B. DE-OS 22 01 432, DE-OS 29 04 490 und DE-OS 30 38 598). Der technische Nachteil einer mehrstufigen Reaktion liegt auf der Hand.

Ferner ist bekannt, dass man aus Glykolsäuremethylester und Dimethylamin bei 35° C und einer Reaktionsdauer von einer Woche Glykolsäure-N,N-dimethylamid erhält. Ein Nachteil dieses Verfahrens ist jedoch die lange Reaktionszeit, die einer technischen Anwendbarkeit entgegensteht. Ein weiterer, grösserer Nachteil liegt in der Beschränkung der als Ausgangsprodukte verwendbaren Amine. Schon Diethylamin lässt sich unter diesen Reaktionsbedingungen nicht mehr umsetzen [vgl. J. org. Chemistry *15*, 323 (1950)].

Es ist auch eine Methode zur Synthese von bestimmten Glykolsäureamiden bekannt geworden, bei welcher man Diglykolid oder Polyglykolide mit entsprechenden Aminen mehrere Stunden erhitzt und anschliessend destillativ aufarbeitet. Das hierfür benötigte Diglykolid bzw. Polyglykolid wird durch Erhitzen von Natriumchloracetat, gegebenenfalls in Gegenwart von Kupfer, erzeugt und durch Destillation isoliert (vgl. J. Prakt, Chem. *18* (1962), S. 141-149; DE-OS 32 22 229).

Auch dass die direkte Umsetzung von Glykolsäureestern mit entsprechenden Aminen unter bestimmten Bedingungen zu den gewünschten Glykolsäureamiden führt, ist bekannt (vgl. DE-OS 32 44 956). Der Nachteil dieses wie auch des vorhergehenden Verfahrens ist die Beschränkung auf bestimmte, insbesondere aliphatische Amine. Aromatische Aminoverbindungen lassen sich nach diesen Verfahren aufgrund ihrer geringeren Basizität nicht umsetzen. Auch die im letzteren Fall erforderliche Herstellung der Glykolsäureester als zusätzlich notwendige Synthesestufe stellt einen erheblichen Nachteil der Methode dar.

Es wurde nun gefunden, dass man Glykolsäureamide der allgemeinen Formel (I)

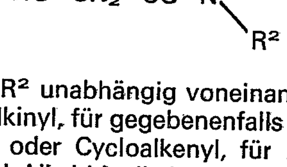 (I)

in welcher

$R^1$ und $R^2$ unabhängig voneinander für Alkyl, Alkenyl, Alkinyl, für gegebenenfalls substituiertes Cycloalkyl oder Cycloalkenyl, für Halogenalkyl, Alkoxyalkyl, Alkylthioalkyl oder Alkoxy, für Aralkyl oder für gegebenenfalls substituiertes Aryl stehen,

oder

$R^1$ und $R^2$ zusammen mit dem Stickstoffatom, an welches sie gebunden sind, für einen gegebenenfalls substituierten, gesättigten oder ungesättigten Heterocyclus stehen, der weitere Heteroatome enthalten kann,

erhält, wenn man Chloracetamide der allgemeinen Formel (II)

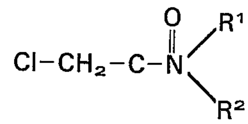 (II)

in welcher

$R^1$ und $R^2$ die oben angegebene Bedeutung haben,

mit Kaliumcarbonat in Gegenwart eines aprotischen Amids als Verdünungsmittel und gegebenenfalls in Gegenwart eines Phasentransfer-Katalysators umsetzt zu symmetrischen Carbonaten der Formel (III)

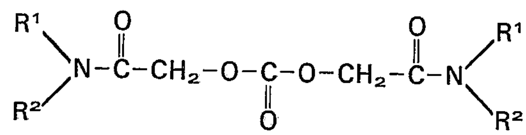 (III)

in welcher

$R^1$ und $R^2$ die oben angegebene Bedeutung haben,

und diese entweder nach vorheriger Isolierung in einer separaten 2. Stufe oder ohne intermediäre Isolierung direkt im Eintopfverfahren durch Umsetzung mit einem primären Alkohol (d.h. durch Umesterung) in Gegenwart eines Alkalimetallhydroxids als Katalysator entacyliert.

Es ist als ausgesprochen überraschend zu bezeichnen, dass die Substitution des Chloridatoms durch eine Hydroxygruppe unter den erfindungsgemässen Reaktionsbedingungen glatt abläuf, da aus dem Stand der Technik bekannt war, dass ein Gemisch aus Kaliumcarbonat und dem im erfindungsgemässen Verfahren vorzugsweise als Verdünungsmittel verwendeten Dimethylformamid alkylierende Eigenschaften besitzt (vgl. z.B. Synthetic Communications *1977*, 559-568) und dass folglich aus intermediär gebildeter Hydroxyverbindung unter den basischen Reaktionsbedingungen Ether entstehen sollten [vgl. auch Tetrahedron Letters *38*, 3251-3254 (1975); C. Ferri, «Reaktionen der organischen Synthese», S. 396, Thieme Verlag Stuttgart 1978; Chem. Ber. *114*, 1210-1215 (1981)]. Die zur glatten Umsetzung zu den gewünschten Glykolsäureamiden erforderliche intermediäre Bildung eines symmetrischen Carbonats der Formel (III)

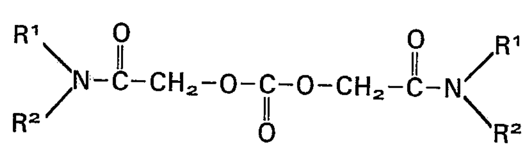 (III)

erfolgt völlig überraschend, da bekannt war, dass eine derartige Carbonatbildung in aprotischen

Verdünungsmitteln nicht zu erwarten war (vgl. z.B. J. Org. Chem. *43*, 4682-4684 (1978); Liebigs Ann. Chem. *1981*, 28-32).

Nach dem erfindungsgemässen Verfahren erhält man vorzugsweise Verbindungen der Formel (I), bei denen

$R^1$ und $R^2$ unabhängig voneinander für geradkettiges oder verzweigtes Alkyl mit 1 bis 8 Kohlenstoffatomen, für jeweils geradkettiges oder verzweigtes Alkenyl und Alkinyl mit jeweils 2 bis 8 Kohlenstoffatomen, oder für jeweils gegebenenfalls einfach oder mehrfach, gleich oder verschieden substituiertes Cycloalkyl oder Cycloalkenyl mit jeweils 3 bis 7 Kohlenstoffatomen stehen, wobei als Substituenten insbesondere Alkylreste mit 1 bis 4 Kohlenstoffatomen infrage kommen, ausserdem für jeweils geradkettiges oder verzweigtes Alkoxy, Alkoxyalkyl und Alkylthioalkyl mit 1 bzw. 2 bis 8 Kohlenstoffatomen, für Halogenalkyl mit 1 bis 8 Kohlenstoffatomen und 1 bis 5 Halogenatomen (insbesondere Fluor, Chlor und Brom), für Aralkyl mit 6 bis 10 Kohlenstoffatomen im Arylteil und 1 bis 2 Kohlenstoffatomen im Alkylteil, sowie für gegebenenfalls einfach oder mehrfach, gleich oder verschieden substituiertes Aryl mit 6 oder 10 Kohlenstoffatomen stehen, wobei als Substituenten im Arylteil jeweils infrage kommen: Halogen, jeweils geradkettiges oder verzweigtes Alkyl, Alkoxy oder Alkylthio mit jeweils 1 bis 4 Kohlenstoffatomen, Halogenalkyl, Halogenalkoxy und Halogenalkylthio mit jeweils 1 bis 2 Kohlenstoffatomen und 1 bis 5 Halogenatomen (insbesondere Fluor, Chlor oder Brom), sowie Nitro oder

$R^1$ und $R^2$ zusammen mit dem Stickstoffatom, an das sie gebunden sind, für einen gegebenenfalls einfach oder mehrfach, gleich oder verschieden substituierten gesättigten oder ungesättigten, 5- bis 7-gliedrigen Heterocyclus stehen, der bis zu 2 weitere Heteroatome, insbesondere Stickstoff oder Sauerstoff enthalten kann, wobei als Substituenten infrage kommen: geradkettiges oder verzweigtes Alkyl mit 1 bis 6 Kohlenstoffatomen, auch in Form eines anellierten Ringsystems, Aryl mit 6 oder 10 Kohlenstoffatomen, auch in Form eines anellierten Ringsystems, oder Dioxyalkylen mit 2 bis 3 Kohlenstoffatomen.

Das erfindungsgemässe Verfahren betrifft besonders bevorzugt Verbindungen der Formel (I), in welcher

$R^1$ und $R^2$ unabhängig voneinander für geradkettiges oder verzweigtes Alkyl mit 1 bis 6 Kohlenstoffatomen, für jeweils geradkettiges oder verzweigtes Alkenyl oder Alkinyl mit jeweils 2 bis 6 Kohlenstoffatomen, für jeweils gegebenenfalls ein- bis dreifach, gleich oder verschieden durch Methyl oder Ethyl substituiertes Cycloalkyl bzw. Cycloalkenyl mit 5 bis 7 Kohlenstoffatomen, für jeweils verzweigtes oder geradkettiges Alkoxy, Alkoxyalkyl oder Alkylthioalkyl mit 1 bis 6 Kohlenstoffatomen, für Halogenalkyl mit 1 bis 6 Kohlenstoffatomen und 1 bis 5 Halogenatomen (insbesondere Fluor, Brom und Chlor), für Benzyl sowie für gegebenenfalls ein- bis dreifach gleich oder verschieden substituiertes Phenyl stehen, wobei als Substituenten besonders bevorzugt sind: Methyl, Ethyl, Methoxy, Methylthio, Trifluormethyl, Trifluormethoxy, Trifluormethylthio, Fluor, Chlor oder Nitro oder

$R^1$ und $R^2$ zusammen mit dem Stickstoffatom, an das sie gebunden sind, für einen gegebenenfalls ein- bis dreifach, gleich oder verschieden substituierten Heterocyclus der Formel

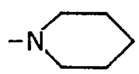 ; 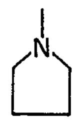 ; 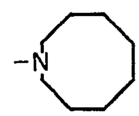 ; 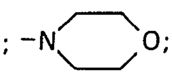 ;

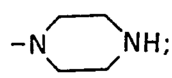 ; 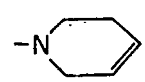 ; 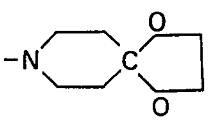 ;

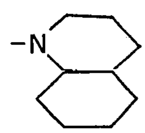 ; 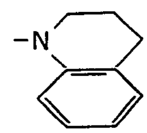 ; 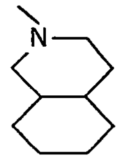 ; 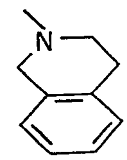

stehen, wobei als Substituenten besonders bevorzugt sind: Methyl, Ethyl und Phenyl.

Als Beispiele für die Verbindungen der Formel (I) seien genannt:

Glykolsäure-dimethylamid, -diethylamid, di-n-propylamid, diiso-propylamid, -di-n-butylamid, -diisobutylamid, -N-methyl-N-n-propylamid, -N-methyl-N-n-butylamid, -N-methyl-N-iso-propylamid, -N-methyl-N-isobutylamid, -N-methyl-N-t-butylamid, -N-methyl-N-sek.-butylamid, -N-ethyl-N-n-propylamid, -N-ethyl-N-iso-propylamid, -N-ethyl-N-n-butylamid, -N-ethyl-N-isobutylamid, -N-ethyl-sek.-butylamid, -N-ethyl-tert.-butylamid, -N-n-propyl-N-iso-propylamid, -N-n-propyl-N-n-butylamid, -N-n-propyl-N-isobutylamid, -N-n-propyl-N-sek.-butylamid, -N-n-propyl-N-tert.-butylamid, -N-n-butyl-N-isobutylamid, N-n-butyl-N-sek.-butylamid, -N-n-butyl-N-tert.-butylamid, -di-(2-methoxyethyl)-amid, -diallylamid, -N-methyl-N-propargylamid, -N-methyl-N-(1-methylpropargyl)-amid, -dipropargylamid, -N-methyl-N-cyclopentylamid, -N-methyl-N-cyclohexylamid, -N-methyl-N-(1,1-dimethylpropargyl)-amid, -N-methyl-N-(2,2,2-trifluorethyl)-amid, -N-ethyl-N-cyclohexylamid, -N-methylanilid, -N-ethylanilid, -N-propylanilid, -N-isopropylanilid, -N-butylanilid, -N-isobutylanilid, -N-sek.-butylanilid, -N-t-butylanilid, -dibenzylamid, N-methyl-N-benzylamid, -N-ethyl-N-benzylamid, -N-propyl-N-benzylamid, -N-butyl-N-benzyl amid, -pyrrolidid, 2-methylpyrrolidid, -morpholid, -piperidid, -2-methylpiperidid, -4-methylpiperidid, -2,4-dimethylpiperidid, -2,4,6-trimethylpiperidid, -2-ethylpiperidid, -4-ethylpiperidid, -2,4-diethylpiperidid, -2,4,6-triethylpiperidid, -2-methyl-4-ethylpiperidid, -2-ethyl-4-methylpiperidid, -2-methyl-5-ethylpiperidid, -2-ethyl-5-methylpiperidid, -2-methyl-6-ethylpiperidid, -1,2,3,4-tetrahydrochinolid, -2-methyl-1,2,3,4-tetra-hydrochinolid, -perhydrochinolid, -2-methylperhydrochinolid, -3-methylpiperidid, -3,5-dymethylpiperidid, -3,5-diethylpiperidid, -N-methyl-N-methoxymethylamid, -N-methyl-N-cyclohex-1-enylamid, -N-methyl-N-(3,5,5-trimethylcyclohex-1-enyl)-amid, -6-methylperhydrochinolid, -N-benzylanilid, -N-benzyl-N-propylamid, -N-ethyl-N-(2,2,2-trifluorethyl)-amid, -N-N-di-(2-methoxyethyl)-amid, -N-methoxy-N-butylamid, -N-methyl-N-(2-methyl-perhydrofuran-2-yl)-methylamid, -perhydroazepid, -3-ethyl-piperidid, -4-phenyl-1,4-piperazid, -3,3,5-trimethylperhydroazepid, -3,4-dioxyethylenpiperidid, -3-methyl-morpholid, -3,5-dymethylmorpholid, -4-methyl-1,4-piperazid, -3,4-dehydropiperid und -1,2,3,4-tetrahydroisochinolid.

Verwendet man beispielsweise N-Methylchloracetanilid als Ausgangsverbindung, Dimethylformamid als Verdünnungsmittel und ein Gemisch aus Triethylamin und Kaliumbromid als Katalysator für die 1. Stufe, sowie Methanol als primären Alkohol (und Verdünnungsmittel) und Kaliumhydroxid als Katalysator für die 2. Stufe, so lässt sich der Reaktionsablauf nach dem erfindungsgemässen Verfahren durch das folgende Formelschema darstellen:

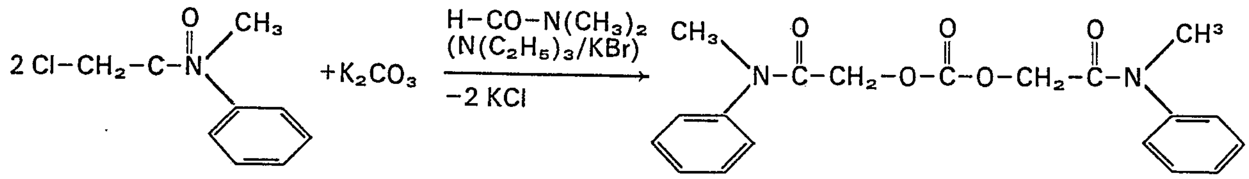


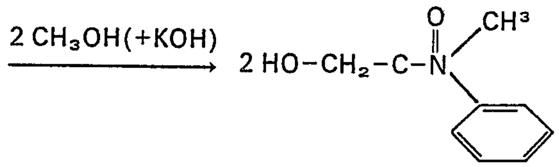


Die zur Durchführung des erfindungsgemässen Verfahrens als Ausgangsstoffe benötigten Chloracetamide sind durch die Formel (II) allgemein definiert. In dieser Formel (II) stehen $R^1$ und $R^2$ vorzugsweise für diejenigen Reste, die bereits im Zusammenhang mit der Beschreibung der erfindungsgemässen Stoffe der Formel (I) als bevorzugt für diese Substituenten genannt wurden.

Die Chloracetamide der Formel (II) sind allgemein bekannte Verbindungen (vgl. z.B. DE-OS 29 04 490).

Das erfindungsgemässe Verfahren wird in Gegenwart eines aprotischen Amids als Verdünnungsmittel durchgeführt. Vorzugsweise verwendet man N,N-Dimethylacetamid, N,N-Dimethylformamid, N-Methylformanilid, N-Methylpyrrolidon oder N-Methylpiperidon. Von besonderem Vorteil ist dabei, dass diese Verdünnungsmittel nicht in wasserfreier Form, sondern in Form der technischen Lösungsmittel (d.h. mit einem Wassergehalt bis zu ca. 3%) eingesetzt werden können.

Das erfindungsgemässe Verfahren kann vorteilhaft in Gegenwart eines geeigneten Phasentransfer-Katalysators durchgeführt werden. Besonders geeignet sind übliche Phasentransferkatalysatoren wie beispielsweise Tetrabutylammoniumiodid, Tetrabutylammoniumbromid, Tributylmethylphosphoniumbromid, Trimethyl-$C_{13}/C_{15}$-alkylammoniumchlorid, Dibenzyldimethylammoniummethylsulfat, Dimethyl-$C_{12}/C_{14}$-alkylbenzylammoniumchlorid, Tetrabutylammoniumhydroxid, 18-Krone-6, Triethylbenzylammoniumchlorid, Trimethylbenzylammoniumchlorid, oder Substanzen, die im Reaktionsgemisch wirksame Phasentransferkatalysatoren (z.B. substituierte Ammoniumsalze) bilden; hierzu gehören beispielsweise tertiäre Amine (wie Triethylamin) im Gemisch mit Alkalimetallbromiden (wie KBr), welche mit überschüssigem Chloracetamid (I) zu entsprechend substituierten Ammoniumbromiden reagieren.

Die Reaktionstemperaturen können bei der Durchführung der 1. Stufe des erfindungsgemässen Verfahrens in einem grösseren Bereich variiert werden. Im allgemeinen arbeitet man bei Temperaturen zwischen 20° C und 150° C, vorzugsweise bei Temperaturen zwischen 60° C und 110° C.

Zur Durchführung der 2. Stufe des erfindungsgemässen Verfahrens (Entacylierung durch Umesterung) ist es erforderlich, einen primären Alkohol zuzusetzen. Besonders geeignet hierzu sind Methanol oder Ethanol.

Die 2. Stufe des erfindungsgemässen Verfahrens erfordert die Gegenwart eines Alkalimetalhydroxids als Katalysator. Besonders geeignet sind Natriumhydroxid oder Kaliumhydroxid.

Die Reaktionstemperaturen können bei der Durchführung der 2. Stufe des erfindungsgemässen Verfahrens ebenfalls in einem grösserem Bereich variiert werden. Im allgemeinen arbeitet man bei Temperaturen zwischen 40° C und 120° C, vorzugsweise bei Temperaturen zwischen 60° C und 110° C.

Beide Stufen des erfindungsgemässen Verfahrens werden im allgemeinen bei Normaldruck durchgeführt.

Zur Durchführung des erfindungsgemässen Verfahrens setzt man in der 1. Stufe pro Mol Chloracetamid der Formel (II) im allgemeinen 0,5 bis 1,3 Mol, vorzugsweise 0,8 bis 1,0 Mol Kaliumcarbonat und 0,01 bis 0,5 Mol, vorzugsweise 0,03 bis 0,3 Mol Phasentransferkatalysator sowie in der 2. Stufe mindestens 2 Mol, vorzugsweise 10 bis 20 Mol, primären Alkohol und 0,001 bis 0,01 Mol Alkalimetallhydroxid ein. Der Alkoholüberschuss dient in der 2. Stufe als Verdünnungsmittel.

Dazu stellt man zunächst eine Suspension aus Kaliumcarbonat und Phasentransferkatalysator in dem als Verdünnungsmittel verwendeten aprotischen Amid hier, setzt dann das Chloracetamid der Formel (II) zu und rührt die Mischung mehrere Stunden bei der erforderlichen Temperatur.

Zur Isolierung der intermediär gebildeten Carbonate der Formel (III) filtriert man ungelöste Bestandteile aus der noch heissen Reaktionsmischung ab und entfernt aus dem Filtrat das Lösungsmittel destillativ. Die erhaltenen Kristalle werden mit Methanol gewaschen und eventuell aus einem geeigneten Lösungsmittel umkristallisiert.

Zur Spaltung der Carbonate der Formel (III) (2. Stufe/Entacylierung) rührt man diese mit den erforderlichen Mengen an Katalysator und primärem Alkohol 1 bis 3 Stunden bei der erforderlichen Temperatur, filtriert ungelöste Bestandteile ab, entfernt den überschüssigen Alkohol im Vakuum und isoliert die Endprodukte der Formel (I) durch Kristallisation oder Destillation.

Bei der einstufigen Reaktionsführung (Eintopfverfahren) setzt man der Reaktionsmischung aus Chloracetamid, Kaliumcarbonat, Phasentransferkatalysator und dem als Verdünnungsmittel verwendeten aprotischen Amid, nachdem man sie für mehrere Stunden bei erhöhter Temperatur gerührt hat, die erforderliche Menge an Alkalimetallhydroxid und primärem Alkohol zu und erhitzt für eine weitere Stunde auf die erforderliche Temperatur. Die Aufarbeitung und Isolierung der Glykolsäureamide der Formel (I) erfolgt wie bei der zweistufigen Reaktionsführung beschrieben.

In einer besonderen Ausführungsform der «einstufigen» Reaktionsführung des erfindungsgemässen Verfahrens (wobei die Carbonate (III) nicht isoliert werden) ist es auch möglich, die als Ausgangsverbindungen verwendeten Chloracetamide der Formel (II) in einer vorgelagerten Reaktion in demselben «Eintopf» durch Umsetzung von Chloracetylchlorid mit einem Amin der Formel (IV)

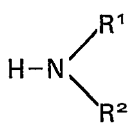

(IV)

in welcher

$R^1$ und $R^2$ die oben angegebene Bedeutung haben,

in Gegenwart von einem zusätzlichen Mol an Kaliumcarbonat sowie in Gegenwart des bei der 1. Stufe des erfindungsgemässen Verfahrens als Verdünnungsmittel verwendeten aprotischen Amids bei Temperaturen zwischen 0° und 30° C herzustellen (vgl. auch Herstellungsbeispiele).

Die nach dem erfindungsgemässen Verfahren herstellbaren bekannten Glykolsäureamide der Formel (I) sind wichtige Zwischenprodukte zur Synthese von Pflanzenschutzmitteln, wie z.B. den herbizid wirksamen Heteroaryloxyacetamiden (vgl. z.B. EP 5 501, EP 18 497, DE-OS 3 038 599, DE-OS 3 038 608, DE-OS 3 038 635, DE-OS 3 038 652, EP 37 524, EP 37 525, EP 37 526, EP 37 527, EP 37 938, US 3 399 988, DE-OS 2 201 432, DE-OS 2 647 568).

*Herstellungsbeispiele:*

*Beispiel 1:*

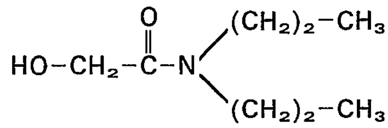

(I-1)

(einstufige Reaktionsführung)

Zu einer Suspension aus 200 ml Dimethylformamid, 69,1 g (0,5 Mol) Kaliumcarbonat und 5 ml Triethylamin tropft man unter Rühren bei 60° C eine Lösung aus 92,3 g (0,52 Mol) N,N-Di-n-propyl-chloracetamid in 100 ml Dimethylformamid. Nach beendeter Zugabe rührt man weitere 3 Stunden bei 100° bis 110° C, kühlt auf 50° C ab, gibt 0,2 g (0,0035 Mol) Kaliumhydroxid und 150 ml Methanol zu und erhitzt für eine Stunde auf Rückfluss-Temperatur. Zur Aufarbeitung filtriert man unlösliche Bestandteile ab, entfernt das Lösungsmittel im Vakuum und destilliert den Rückstand im Hochvakuum. Man erhält 66 g (80% der Theorie) an N,N-Di-n-propylglykolsäureamid vom Siedepunkt Kp 110° C bei, 2,6 mbar mit einem gaschromatographisch bestimmten Gehalt von 95%.

In entsprechender Weise und gemäss den allgemeinen Angaben zur Herstellung erhält man die folgenden Glykolsäureamide der allgemeinen Formel (I):

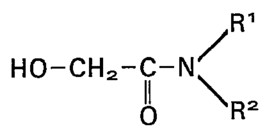

(I)

*Tabelle 1*

| Beispiel Nr. | $R^1$ | $R^2$ | Siedepunkt | Ausbeute (%) |
|---|---|---|---|---|
| I-2 | $CH_3$ | $C_6H_5$ | 107-109° C/ 0,8 mbar | 69 |
| I-3 | $C_2H_5$ | $C_2H_5$ | 113-114° C/16 mbar | 80 |
| I-4 | $CH_2=CH-CH_2-$ | $CH_2=CH-CH_2-$ | 85° C/1,3 mbar | 65 |
| I-5 | $-CH_2-CH_2-CH_2-CH_2-CH_2-CH_2-$ | | 102° C/1,7 mbar | 78 |

*Beispiel 2:*

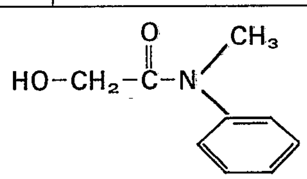

(I-2) (zweistufige Reaktionsführung)

*1. Stufe*

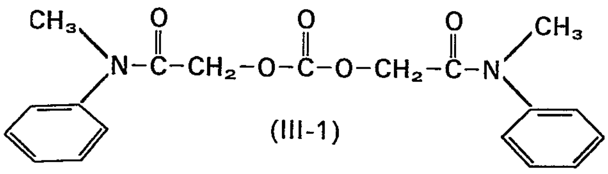

(III-1)

Zu einer Suspension aus 100 ml Dimethylformamid, 27,6 g (0,2 Mol) Kaliumcarbonat, 1 g Kaliumbromid und 2 ml Triethylamin gibt man bei 60° C tropfenweise unter Rühren eine Lösung aus 27,6 g (0,2 Mol) N-Methylchloracetanilid in 50 ml Dimethylformamid und rührt nach beendeter Zugabe weitere 3 Stunden bei 100° bis 110° C.

Die auf 70° C abgekühlte Reaktionsmischung wird abgesaugt, das Filtrat im Vakuum eingeengt und der kristalline Rückstand mit Methanol gewaschen, abgesaugt und getrocknet. Man erhält 19,6 g (55% der Theorie) an O,O-Bis-(N-methyl-N-phenylaminocarbonylmethyl)-carbonat vom Schmelzpunkt 167° C.

In entsprechender Weise und gemäss den allgemeinen Angaben zur Herstellung erhält man die folgenden symmetrischen Carbonate der allgemeinen Formel (III):

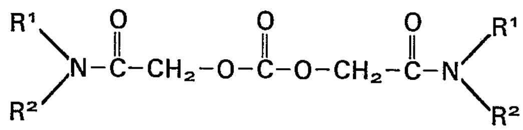

(III)

*Tabelle 2*

| Beispiel Nr. | $R^1$ | $R^2$ | Siedepunkt (° C) | Ausbeute (%) |
|---|---|---|---|---|
| III-2 | $CH_3-(CH_2)_2-$ | $CH_3-(CH_2)_2-$ | 51 | 44 |
| III-3 | $CH_2=CH-CH_2-$ | $CH_2=CH-CH_2-$ | 55-56 | 28 |
| III-4 | $-CH_2-CH_2-CH_2-CH_2-CH_2-CH_2-$ | | 148 | 26 |
| III-5 | $-CH_2-CH_2-O-CH_2-CH_2-$ | | 227,5 | 22 |

*2. Stufe*

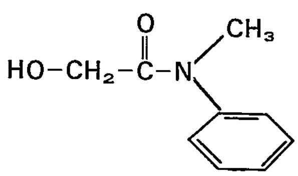

(I-2)

35,6 g (0,1 Mol) O,O-Bis-(N-methyl-N-phenylaminocarbonyl-methyl)-carbonat werden in 80 ml Methanol mit 0,1 g Kaliumhydroxid versetzt und eine Stunde bei Rückfluss-Temperatur gerührt. Nach Abfiltrieren der unlöslichen Bestandteile und Entfernen des Lösungsmittels im Vakuum erhält man 33,3 g (99% der Theorie) an Glykolsäure-N-methylanilid vom Schmelzpunkt 52-53° C und einem gaschromatographisch bestimmten Gehalt von 98%.

*Beispiel 3:*

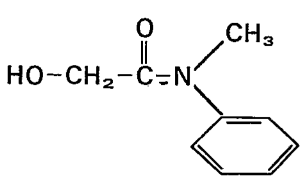

(I-2)

(Eintopfverfahren über 3 Stufen)

Zu einer Suspension aus 300 ml Dimethylformamid, 53,5 g (0,5 Mol) N-Methylanilin und 69,1 g (0,5 Mol) Kaliumcarbonat gibt man bei 20° bis 25° C unter Kühlung tropfenweise 57,6 g (0,51 Mol) Chloracetylchlorid. Nach beendeter Zugabe rührt man weitere 90 Minuten (bis zum Ende der Gasentwicklung) bei Raumtemperatur nach, gibt dann weitere 69,1 g (0,5 Mol) Kaliumcarbonat, 3 g Kaliumbromid und 5 ml Triethylamin zu und erhitzt für 2 Stunden auf 100° bis 110° C. Zu der auf 50° C abgekühlten Reaktionsmischung gibt man 150 ml Methanol und 0,2 g Kaliumhydroxid und erwärmt für eine weitere Stunde auf Rückfluss-Temperatur. Die erkaltete Reaktionsmischung wird abgesaugt und das Filtrat im Vakuum eingeengt. Man erhält 81 g eines öligen Rohproduktes mit einem gaschromatographisch bestimmten Gehalt von 61,2% an Glykolsäure-N-methyl-anilid (I-2) (entsprechend einer Ausbeute von 60% der Theorie, bezogen auf N-Methylanilin).

In entsprechender Weise erhält man in einem Eintopfverfahren über 3 Stufen N,N-Di-n-propyl-glykolsäureamid:

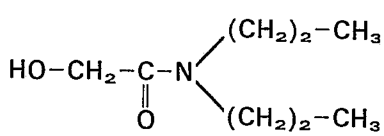

(I-1)

Ausbeute: 67% der Theorie, bezogen auf Di-n-propylamin;

Siedepunkt 80°-85° C bei 0,5 mbar.

## Patentansprüche

1. Verfahren zur Herstellung von Glykolsäureamiden der allgemeinen Formel (I)

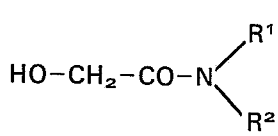

(I)

in welcher

$R^1$ und $R^2$ unabhängig voneinander für Alkyl, Alkenyl, Alkinyl, für gegebenenfalls substituiertes Cycloalkyl oder Cycloalkenyl, für Halogenalkyl, Alkoxyalkyl, Alkylthioalkyl oder Alkoxy, für Aralkyl oder für gegebenenfalls substituiertes Aryl stehen, oder

$R^1$ und $R^2$ zusammen mit dem Stickstoffatom, an welches sie gebunden sind, für einen gegebenenfalls substituierten, gesättigten oder ungesättigten Heterocyclus stehen, der weitere Heteroatome enthalten kann,

dadurch gekennzeichnet, dass man Chloracetamide der allgemeinen Formel (II)

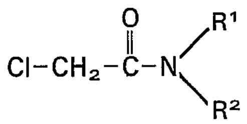 (II)

in welcher

$R^1$ und $R^2$ die oben angegebene Bedeutung haben,

mit Kaliumcarbonat in Gegenwart eines aprotischen Amids als Verdünnungsmittel und gegebenenfalls in Gegenwart eines Phasentransfer-Katalysators umsetzt zu symmetrischen Carbonaten der Formel (III)

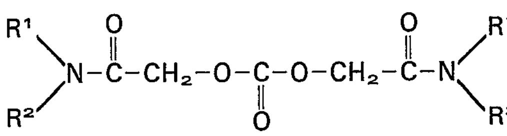 (III)

in welcher

$R^1$ und $R^2$ die oben angegebene Bedeutung haben,

und diese entweder nach vorheriger Isolierung in einer separaten 2. Stufe oder ohne intermediäre Isolierung direkt im Eintopfverfahren durch Umsetzung mit einem primären Alkohol (d.h. durch Umesterung) in Gegenwart eines Alkalimetallhydroxids als Katalysator entacyliert.

2. Verfahren nach Anspruch 1, dadurch gekennzeichnet, dass man die erste Reaktionsstufe bei Temperaturen zwischen 20° und 150° C durchführt.

3. Verfahren nach Anspruch 1, dadurch gekennzeichnet, dass man die zweite Reaktionsstufe bei Temperaturen zwischen 40° und 120° C durchführt.

4. Verfahren nach Anspruch 1, dadurch gekennzeichnet dass man in der ersten Reaktionsstufe als aprotisches Amid Dimethylformamid einsetzt.

5. Verfahren nach Anspruch 1, dadurch gekennzeichnet, dass man in der zweiten Reaktionsstufe als primären Alkohol Methanol oder Ethanol einsetzt.

6. Verfahren nach Anspruch 1, dadurch gekennzeichnet, dass man in der ersten Reaktionsstufe auf 1 Mol Chloracetamid (II) 0,5 bis 1,3 Mol Kaliumcarbonat und 0,01 bis 0,5 Mol Phasentransfer-Katalysator und in der zweiten Reaktionsstufe mindestens 2 Mol, vorzugsweise 10-20 Mol, primären Alkohol und 0,001 bis 0,01 Mol Alkalihydroxid einsetzt.

7. Ausgestaltung des Verfahrens nach Anspruch 1, dadurch gekennzeichnet, dass die Herstellung der Chloracetamide (II) in einer vorgelegten Reaktion durch Umsetzung von Chloracetylchlorid mit einem Amin der Formel

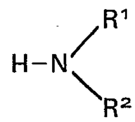 (IV)

in welcher

$R^1$ und $R^2$ die in Anspruch 1 genannte Bedeutung haben,

mit der ersten und zweiten Reaktionsstufe des Verfahrens gemäss Anspruch 1, wobei die Carbonate (III) nicht isoliert werden, zu einem «Eintopfverfahren» zusammengefasst wird.

## Claims

1. Process for the preparation of glycolamides of the general formula (I)

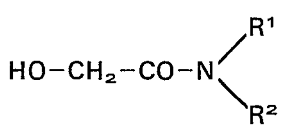 (I)

in which

$R^1$ and $R^2$ independently of one another represent alkyl, alkenyl, alkinyl, optionally substituted cycloalkyl or cycloalkenyl, halogenoalkyl, alkoxyalkyl, alkylthioalkyl or alkoxy, aralkyl or optionally substituted aryl, or

$R^1$ and $R^2$, together with the nitrogen atom to which they are attached, represent an optionally substituted, saturated or unsaturated heterocyclic structure which can contain further heteroatoms,

characterized in that chloroacetamides of the general formula (II)

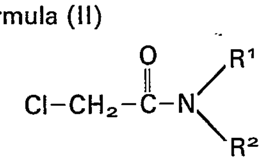 (II)

in which

$R^1$ and $R^2$ have the meaning indicated above, are reacted with potassium carbonate in the presence of an aprotic amide as a diluent and, if appropriate, in the presence of a phase transfer catalyst to give symmetrical carbonates of the formula (III)

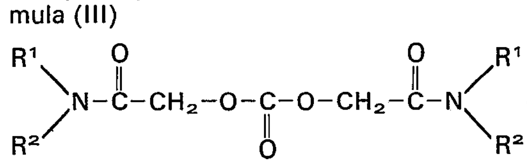 (III)

in which

$R^1$ and $R^2$ have the meaning indicated above, and these carbonates are deacylated, either after prior isolation in a separate 2nd stage or, without intermediate isolation, directly in a one-pot process, by reaction with a primary alcohol (that is to say by transesterification) in the presence of an alkali metal hydroxide as catalyst.

2. Process according to Claim 1, characterized in that the first reaction stage is carried out at temperatures between 20° and 150° C.

3. Process according to Claim 1, characterized in that the second reaction stage is carried out at temperatures between 40° and 120° C.

4. Process according to Claim 1, characterized in that dimethylformamide is employed as the aprotic amide in the first reaction stage.

5. Process according to Claim 1, characterized in that methanol or ethanol is employed as the primary alcohol in the second reaction stage.

6. Process according to Claim 1, characterized in that 0.5 to 1.3 Mol of potassium carbonate and 0.01 to 0.5 Mol of phase transfer catalyst are employed for 1 Mol of chloroacetamide (II) in the first reaction stage, and at least 2 Mol, preferably 10-20 Mol, of primary alcohol and 0.001 to 0.01 Mol of alkali metal hydroxide are employed in the second reaction stage.

7. Embodiment of the process according to Claim 1, characterized in that the preparation of the chloroacetamides (II) in a prior reaction by reacting chloroacetyl chloride with an amine of the formula

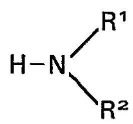 (IV)

in which

$R^1$ and $R^2$ have the meaning mentioned in Claim 1, is combined to form a "one-pot process" with the first and second reaction stages of the process according to Claim 1, the carbonates (III) not being isolated.

**Revendications**

1. Procédé de production d'amides d'acide glycolique de formule générale (I)

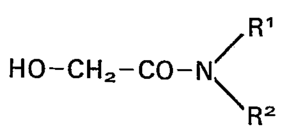 (I)

dans laquelle

$R^1$ et $R^2$ représentent, indépendamment l'un de l'autre, un groupe alkyle, alcényle, alcynyle, un groupe cycloalkyle ou cycloalcényle éventuellement substitué, un groupe halogénalkyle, alkoxyalkyle, alkylthioalkyle ou alkoxy, un groupe aralkyle ou un groupe aryle éventuellement substitué, ou bien

$R^1$ et $R^2$ forment conjointement avec l'atome d'azote auquel ils sont liés un hétérocycle saturé ou non saturé éventuellement substitué, qui peut contenir d'autres hétéroatomes, caractérisé en ce qu'on fait réagir des chloracétamides de formule générale (II)

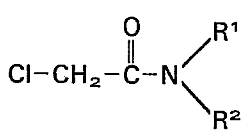 (II)

dans laquelle

$R^1$ et $R^2$ ont la définition indiquée ci-dessus, avec le carbonate de potassium en présence d'un amide aprotique comme diluant et, le cas échéant, en présence d'un catalyseur de transfert de phase pour former des carbonates symétriques de formule (III)

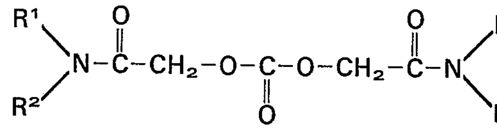 (III)

dans laquelle

$R^1$ et $R^2$ ont la définition donnée ci-dessus, et en désacylant ces carbonates, soit après un isolement préalable dans une seconde étape séparée, soit sans isolement intermédiaire, directement dans le procédé en réacteur unique par réaction avec un alcool primaire (c'est-à-dire par transestérification) en présence d'un hydroxyde de métal alcalin comme catalyseur.

2. Procédé suivant la revendication 1, caractérisé en ce qu'on conduit la première étape de la réaction à des températures comprises entre 20° et 150° C.

3. Procédé suivant la revendication 1, caractérisé en ce qu'on conduit la seconde étape de la réaction à des températures comprises entre 40° et 120° C.

4. Procédé suivant la revendication 1, caractérisé en ce qu'on utilise dans la première étape de la réaction le diméthylformamide comme amide aprotique.

5. Procédé suivant la revendication 1, caractérisé en ce qu'on utilise dans la seconde étape de la réaction le méthanol ou l'éthanol comme alcool primaire.

6. Procédé suivant la revendication 1, caractérisé en ce qu'on utilise dans la première étape de la réaction, par mole de chloracétamide (II), 0,5 à 1,3 mole de carbonate de potassium et 0,01 à 0,5 mole de catalyseur de transfert de phase et, dans la seconde étape de la réaction, au moins 2 moles, de préférence 10 à 20 moles d'alcool primaire et 0,001 à 0,01 mole d'hydroxyde alcalin.

7. Développement du procédé suivant la revendication 1, caractérisé en ce que la production des chloracétamides (II) dans une réaction préliminaire du chlorure de chloracétyle avec une amine de formule

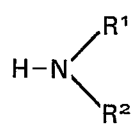 (IV)

dans laquelle

$R^1$ et $R^2$ ont la définition mentionnée dans la revendication 1,

est groupée en un «procédé en réacteur unique» avec la première et la seconde étape de réaction du procédé suivant la revendication 1, les carbonates (III) n'étant alors pas isolés.